# EUROPEAN PATENT SPECIFICATION

(11) **EP 3 150 201 B1**
(45) Date of publication and mention of the grant of the patent: **19.02.2020**
(21) Application number: 15815459.1
(22) Date of filing: 30.06.2015
(51) Int. Cl.: A61K 9/48, A61K 9/28, A61K 47/32, A61K 47/30, A61K 45/06

(54) **COMPOSITE PREPARATION COMPRISING 5- -REDUCTASE INHIBITOR-CONTAINING FILM COATING LAYER, AND METHOD FOR PRODUCING THE COMPOSITE PREPARATION**
VERBUNDSTOFFPRÄPARAT MIT 5-REDUKTASE-INHIBITORHALTIGER FILMBESCHICHTUNGSSCHICHT UND VERFAHREN ZUR HERSTELLUNG DES VERBUNDSTOFFPRÄPARATS
PREPARATION COMPOSITE COMPRENANT UNE COUCHE DE REVÊTEMENT PELLICULAIRE CONTENANT UN INHIBITEUR DE LA 5-ALPHA-RÉDUCTASE, ET PROCÉDÉ POUR PRODUIRE LA PRÉPARATION COMPOSITE

(30) Priority: 30.06.2014 KR 20140081223
(43) Date of publication of application: 05.04.2017
(73) Proprietor: Hanmi Pharm. Co., Ltd., Hwaseong-si, Gyeonggi-do 18536 (KR)
(72) Inventor: KIM, Hyung Seo, Hwaseong-si Gyeonggi-do 445-925 (KR); CHO, Jung Hyun, Hwaseong-si Gyeonggi-do 445-330 (KR); KIM, Jin Cheul, Seoul 150-754 (KR); KIM, Yong Il, Suwon-si Gyeonggi-do 440-730 (KR); PARK, Jae Hyun, Suwon-si Gyeonggi-do 443-737 (KR); WOO, Jong Soo, Suwon-si Gyeonggi-do 440-710 (KR)
(74) Representative: Marks & Clerk (Luxembourg) LLP
(86) International application number: PCT/KR2015/006742
(87) International publication number: WO 2016/003180

(56) References cited:
- WO-A1-2014/017833
- WO-A1-2014/182003
- WO-A2-2012/127495
- KR-A- 20080 007 252
- KR-A- 20140 013 436
- US-A1- 2013 171 199
- US-A1- 2013 171 199
- US-A1- 2014 010 872
- BASF , PHARMA INGREDIENTS & SERVICES: 'Kollicoat Protect, 03_040903e-08' TECHNICAL INFORMATION February 2012, pages 1 - 10, XP055380857 Retrieved from the Internet: <URL:http://www.pharma-ingredients.basf.com /Statements/Technical%20 Informations/EN/Pharma%20Solutions/03_04090 3e_Kollicoa2%20Protect.pdf>

## Description

### TECHNICAL FIELD

The present disclosure relates to a composite preparation including a 5-α-reductase inhibitor-containing film coating layer and a method of preparing the same, and more particularly, to a composite preparation including a 5-α-reductase inhibitor-containing film coating layer which may have improved content uniformity, and a high dissolution rate of the active ingredient, and may remain shape stable against common external impacts, and a method of preparing the composite preparation.

### BACKGROUND ART

The prostate gland of a healthy young man is about the size of a walnut, and gradually grows with age. Prostatic hypertrophy is a disease from severe enlargement of the prostate gland by which the urethra passing through the prostate is pressed, leading to various symptoms.

Although not clearly identified yet, the cause of prostatic hypertrophy is known to be various complex factors, as in other chronic diseases. A currently recognized cause of prostatic hypertrophy is aging of normal testicles. The prostate gland is an androgenic hormone (male sex hormone)-dependent organ, which needs continuous supply of the androgenic hormone for normal growth and functions. The prostate gland may shrink if the androgenic hormone is not produced any longer due to castration.

Symptoms of prostatic hypertrophy include lower urinary tract symptoms (LUTS), including symptoms caused by bladder storage disorders, such as frequent urination of eight times or more a day, nocturia, urgent urination accompanied by a strong, sudden sensation to urinate and compelling urge to urinate, urgent urinary incontinence, and painful urination (dysuria); and symptoms caused by bladder discharge disorders, such as retarded urination (in which the patient must wait for urination to begin), interrupted urination (with an intermittent stream of urine), stress urination (in which the patient must apply pressure for urination to begin), weak urinary stream, a sensation of incomplete emptying, and urinary retention.

5-α-reductase inhibitors are drugs typically used to treat prostatic hypertrophy, which may be used alone, and is also known to be used together with tamsulosin or a phosphodiesterase-5 inhibitor (for example, tadalafil, vardenafil, udenafil, or sildenafil) for more effective treatment of prostatic hypertrophy.

5-α-reductase inhibitors may inhibit the conversion of testosterone to dihydrotestosterone which further enhances the enlargement of the prostate gland, and thus may block the enlargement of the prostate and relieve the urinary tract from physical pressure.

Tamsulosin is a drug which selectively blocks α-adrenoceptors, selectively acting in the urogenital organs, and is known to improve urination rates by relaxing the prostate and the smooth muscle surrounding the bladder, and to alleviate the symptoms of benign prostatic hypertrophy.

Tadalafil (Cialis, Lilly ICOS) and vardenafil (Levitra, GSK), as phosphodiesterase-5 inhibitors, were originally developed as drugs for the treatment of impotency. However, these drugs are increasingly being used for more indications, such as prostatic hypertrophy and overactive bladder (Euro Urol 2008; 1236-44; J Uro 2007;1401-7).

Drug combinations, such as that of a 5-α-reductase inhibitor with tamsulosin, or that of a 5-α-reductase inhibitor with phosphodiesterase-5 inhibitor, have advantages in terms of efficacy enhancement due to the different action mechanisms of the two active ingredients administered in combination (EP 1 501 517; BJU Int. 2006 Apr. 97 Suppl 2:39-43; discussion 44-5).

However, in preparing a composite preparation including two or more active ingredients, such as the drug combinations as described above, problems relating to the stability of the active ingredients may arise due to interaction between the two or more different active ingredients. To ensure stability of such a composite preparation, a composite preparation has been developed which includes an active ingredient-containing core and a film coating layer containing another active ingredient, in which the two active ingredients are separated into different layers (EP 1 830 820; US 6,682,759). US 2013/0171199 discloses an oral dispersible tablet comprising sustained release tamsulosin pellets coated with a drug dispersion comprising finasteride.

In such a composite preparation including a 5-α-reductase inhibitor-containing film coating layer, to ensure sufficient bioavailability and rapid efficacy for oral administration, a high dissolution rate and content uniformity of the 5-α-reductase inhibitor contained in the film coating layer of the composite preparation is required. In addition, if the film coating layer has an insufficient tensile strength, the film coating layer may become separated or broken by usual impacts during storage of the product, such that it may be difficult to ensure sufficient efficacy of the composite preparation and maintain merchantable product quality due to changes in appearance and other properties, eventually leading to the inability to commercialize the preparation as a product.

### DETAILED DESCRIPTION OF THE INVENTION

### TECHNICAL PROBLEM

The present disclosure provides a composite preparation including a 5-α-reductase inhibitor-containing film coating layer, which may have a high dissolution rate and content uniformity of the active ingredient, and stable shape and durability against usual external impacts.

The present disclosure provides a method of preparing the composite preparation including the 5-α-reductase inhibitor-containing film coating layer.

### TECHNICAL SOLUTION

According to an aspect of the present invention, there is provided a composite preparation including: a core containing a first active ingredient; and a film coating layer containing a 5-α-reductase inhibitor, wherein the film coating layer is formed by coating with a film coating solution comprising the 5-α-reductase inhibitor and a film coating material in a mixed solvent of water and 30wt% to 80wt% of an organic solvent with respect to a total weight of the mixed solvent, wherein the film coating material is a combination of a polyvinyl alcohol-polyethylene glycol graft copolymer and polyvinyl alcohol, wherein a weight ratio of the polyvinyl alcohol-polyethylene glycol graft copolymer to the polyvinyl alcohol is 7:3 to 4:6, and wherein the 5-α-reductase inhibitor is selected from the group consisting of finasteride, dutasteride, and any combination thereof.

According to another aspect of the present invention, there is provided a method of preparing the above-described composite preparation, the method including: preparing the core containing the first active ingredient; preparing a 5-α-reductase inhibitor-containing coating solution in which the 5-α-reductase inhibitor and the film coating material are dissolved in the mixed solvent of water and 30wt% to 80wt% of an organic solvent based on a total weight of the mixed solvent; and coating the core with the 5-α-reductase inhibitor-containing coating solution.

### ADVANTAGEOUS EFFECTS

According to the one or more embodiments of the present disclosure, a composite preparation may include a 5-α-reductase inhibitor-containing film coating layer which may ensure a high dissolution rate and content uniformity of the active ingredient contained therein and which may have tensile strength durable against usual external impacts.

Thus, the composite preparation may consistently ensure sufficient bioavailability and rapid action, and may undergo no change in appearance and properties as a result of external impacts, while having high merchantable product quality without loss of efficacy.

Furthermore, due to the inclusion of the 5-α-reductase inhibitor-containing film coating layer, the composite preparation may have a remarkably reduced volume, and thus may be in the form of remarkably reduced size of a tablet or capsule compared to a composite formulation made by simply combining the compositions of individual single preparations, increasing the convenience to the patient when taking the medication.

Finasteride, a typical 5-α-reductase inhibitor, is known to be teratogenic even in a trace amount, and thus a pharmaceutical company must be equipped with a separate independent production line exclusive for finasteride and separate from the existing production lines, so as to prevent finasteride from being incorporated into other medical products during preparation of a finasteride-containing formulation. However, since the composite preparation includes finasteride in the form of a film coating layer, the composite preparation may be prepared just by introducing an additional coating facility. Therefore, the composite preparation may be economically prepared.

Therefore, a composite preparation according to any of the embodiments which includes two or more active ingredients, including a 5-α-reductase inhibitor, may have improved efficacy, merchantable product quality, and economical efficiency, and may also improve patient compliance for the combined administration of drugs, including a 5-α-reductase inhibitor.

### BRIEF DESCRIPTION OF THE DRAWINGS

FIG. 1 is a schematic view illustrating a composite preparation (a) including a 5-α-reductase inhibitor-containing film coating layer coated on a surface of a first active ingredient-containing tablet core, and a composite preparation (b) including a 5-α-reductase inhibitor-containing film coating layer coated on a surface of a first active ingredient-containing hard capsule core;
FIG. 2 illustrates the resulting data and graph of a dissolution test of the composite preparations of Examples 1 to 11 performed using the Paddle method among Dissolution Methods in accordance with the General Tests of the Korean Pharmacopoeia (10th edition);
FIG. 3 illustrates the resulting data and graph of a dissolution test of the composite preparations of Comparative Examples 1 to 8 performed using the Paddle method among Dissolution Methods according to the General Tests of the Korean Pharmacopoeia (10th edition);
FIG. 4 illustrates the results of calculating dissolution rate deviations in the dissolution test of the composite preparations of Examples 1 to 11 and Comparative Examples 1 to 8;
FIG. 5 illustrates the results of X-ray diffractometry of finasteride powder as a raw material, and dried films prepared using film coating solutions of Examples 2 and 7 and Comparative Example 2;
FIG. 6 is scanning electron microscopic (SEM) images of dried films prepared using the film coating solutions of Example 1 and Comparative Examples 2 and 3;
FIG. 7 is a photographic image of composite preparations, one of which is evaluated as an acceptable product (a) and the other as a defective product (b);
FIG. 8 is a graph illustrating defect rates of composite preparations of Comparative Examples 9 to 16, evaluated upon unpacking PTP packages thereof;
FIG. 9 is a graph illustrating defect rates of composite preparations of Examples 12 to 21, evaluated upon unpacking PTP packages thereof;
FIG. 10 is a graph illustrating defect rates with respect to coating materials of the composite preparations of Comparative Examples 9 to 12, evaluated upon unpacking PTP packages thereof after storage for one week at about 60°C and at a relative humidity (RH) of 0%;
FIG. 11 is a graph illustrating dissolution rates of finasteride from the composite preparations of Comparative Examples 9 to 12;
FIG. 12 is a graph illustrating dissolution rates of finasteride from the composite preparations of Examples 13 to 16; and
FIG. 13 is a graph illustrating defect rates with respect to different core types of composite preparations of Examples 14, 22, and 23, evaluated upon unpacking PTP packages thereof.

### MODE FOR INVENTION

Unless otherwise defined, all terms (including technical and scientific terms) used herein have the same meaning as commonly understood by one of ordinary skill in the art to which this invention belongs.

To prepare an effective, stable composite preparation containing a 5-α-reductase inhibitor, the inventors of the present disclosure conducted in-depth research into a 5-α-reductase inhibitor-containing film coating layer which may have a high dissolution rate and content uniformity of the active ingredient, and a strong tensile strength durable against common external impacts, and found as a result that when a film coating solution containing the 5-α-reductase inhibitor and a film coating material is prepared using a mixed solvent of water and about 30wt% to 80wt% of an organic solvent, a film coating layer formed from the film coating solution may have a high dissolution rate and content uniformity of the active ingredient.

An aspect of the present disclosure provides a composite preparation as defined in claim 1 including a core containing a first active ingredient; and a film coating layer containing a 5-α-reductase inhibitor, wherein the film coating layer is formed by coating with a film coating solution including the 5-α-reductase inhibitor and a film coating material in an mixed solvent of water and 30wt% to 80wt% of an organic solvent with respect to a total weight of the mixed solvent.

The core containing the first active ingredient may be any solid pharmaceutical formulation commonly used in the field of pharmaceutics. For example, the core may be in the form of a tablet, a hard capsule, or a soft capsule. However, embodiments are not limited thereto. A filler part of the hard capsule or soft capsule may be in the form of any pharmaceutical formulation commonly used in the field of pharmaceutics, for example, granules, pellets, powders, tablets, liquid, or any combinations thereof.

The core may be about 20 wt% to 99.5 wt% of a total weight of the composite preparation.

The film coating layer containing the 5-α-reductase inhibitor may be on a surface of the core.

The film coating solution as defined in claim 1 is a solution containing the 5-α-reductase inhibitor and the film coating material in a mixed solvent of water and an organic solvent, wherein the amount of the organic solvent is 30wt% to 80wt% of a total weight of the mixed solvent. The mixed solvent may be able to dissolve both the coating material and the 5-α-reductase inhibitor.

The amount of the organic solvent may be a volume sufficient to dissolve both the coating material and the 5-α-reductase inhibitor, and may vary depending on a type of the 5-α-reductase inhibitor. For example, when the 5-α-reductase inhibitor is finasteride, the amount of the organic solvent may be about 120 mg to about 576 mg per 5 mg of finasteride (see Examples 1 to 11 and Test Example 1).

The organic solvent may be any organic solvent which may be used for preparation of a film coating solution. In some embodiments, the organic solvent may be methanol, ethanol, acetone, chloroform, dimethyl sulfoxide (DMSO), or any combination thereof. For example, the organic solvent may be ethanol.

The inventors of the present disclosure prepared composite preparations each having a film coating layer obtained by coating a tablet or capsule core containing a first active ingredient with a film coating solution prepared by mixing a 5-α-reductase inhibitor and a coating material in a mixed solvent including water and a various amount of an organic solvent by weight ratio, and conducted a dissolution test of the 5-α-reductase inhibitor with time. As a result, when a film coating solution containing the film coating material dissolved in a mixed solvent of water and 30wt% to 80wt% of an organic solvent is used, the resulting film coating layer was found to have a remarkably increased dissolution rate of the active ingredient with a significantly low deviation in dissolution rates, as compared with when the amount of the organic solvent is outside the above range (see Test Examples 2 and 3). This seems to be attributed to that the mixed solvent of water and 30wt% to 80wt% of an organic solvent may completely dissolve the film coating material and the 5-α-reductase inhibitor, while a mixed solvent of water and an organic solvent out of the above range may not completely dissolve the film coating material and/or the 5-α-reductase inhibitor (see Test Example 1), and more particularly, seems to be attributed to the 5-α-reductase inhibitor, originally in crystalline form, being changed into an amorphous form during film coating layer formation after completely dissolved in the mixed solution, thereby having increased solubility (see Test Example 4).

5-α-reductase inhibitors such as finasteride or dutasteride, as immediate release tablets or common tablets, are known to have tₘₐₓ of 1 to 2 hours, and thus need to be absorbed fast through high-rate dissolution. A composite preparation according to any of the embodiments may have a remarkably high dissolution rate of a 5-α-reductase inhibitor with a low deviation, and thus may be provided as a persistently effective composite preparation.

In some embodiments, the film coating material is a combination of a polyvinyl alcohol-polyethylene glycol graft copolymer and polyvinyl alcohol. According to experiments performed, when a combination of a polyvinyl alcohol-polyethylene glycol graft copolymer and polyvinyl alcohol was used as a film coating material, it was shown that the film coating layer had improved tensile strength, high enough so as not to be separated or broken during storage by common impacts, and a high dissolution rate of the active ingredient contained in the film coating layer, as compared with when other combinations of film coating materials were used.

In particular, the inventors of the present disclosure prepared composite preparations with a film coating layer including a 5-α-reductase inhibitor and a different coating material being formed on tablet or capsule cores containing a first active ingredient, and conducted a defect test with respect to the different coating materials and a dissolution test of the 5-α-reductase inhibitor with time. As a result, when a polyvinyl alcohol-polyethylene glycol graft copolymer, polyvinyl alcohol, povidone, or hypromellose was used alone as a coating material, a defect rate of the film coating layer of the composite preparation, resulting from factors such as separation from the core or breaking, was as high as 20 to 40%, and was as low as less than 2% only when polyvinyl alcohol was used (see Test Example 5). However, when polyvinyl alcohol was used as the coating material, the composite preparation had a low dissolution rate of the 5-α-reductase inhibitor of less than 75% in 15 minutes, as evaluated using a Dissolution Test according to the General Tests of the *Korean Pharmacopoeia* (see Test Example 8). Accordingly, it was difficult to obtain a film coating layer that ensures both a high tensile strength and a sufficiently high dissolution rate. On the other hand, when a combination of a polyvinyl alcohol-polyethylene glycol graft copolymer and polyvinyl alcohol was used as a coating material, a defect rate of the film coating layer, resulting from such as separation from the core or breaking, was markedly reduced to less than 10% (see Test Examples 6 and 7) and the 5-α-reductase inhibitor had a sufficiently high dissolution rate of about 75% or greater in 15 minutes (see Test Examples 2 and 9), indicating remarkable results ensuring both a low film coating defect rate and a high dissolution rate.

The combination of the polyvinyl alcohol-polyethylene glycol graft copolymer and polyvinyl alcohol is in a weight ratio of 7:3 to 4:6, and in some other embodiments, in a weight ratio of about 6:4.

The polyvinyl alcohol-polyethylene glycol graft copolymer may consist of about 65% to about 85% of a polyvinyl alcohol unit and about 15 to 35% of a polyethylene glycol unit, may contain about 0.01% to about 0.5% of colloid silica, and may have a weight average molecular weight of about 35,000 Daltons to about 55,000 Daltons. The polyvinyl alcohol-polyethylene glycol graft copolymer is commercially available, for example, as Kollicoat® IR (available from BASF), which includes about 75% polyvinyl alcohol units and about 25% polyethylene glycol units, contains about 0.3% of colloid silica, and has a weight average molecular weight of about 45,000 Daltons.

The polyvinyl alcohol, which is a water soluble polymer, may have a molecular weight of about 20,000 Daltons to about 200,000 Daltons, wherein the larger the molecular weight becomes, the viscosity may become higher.

The film coating layer may be about 0.5 parts to about 80 parts by weight with respect to 100 parts by weight of the core.

In some embodiments, the polyvinyl alcohol-polyethylene glycol graft copolymer of the composite preparation may consist of about 65% to about 85% of a polyvinyl alcohol unit and about 15% to about 35% of a polyethylene glycol unit, may contain about 0.01wt% to about 0.5wt% of colloid silica, and may have a weight average molecular weight of about 35,000 Daltons to about 55,000 Daltons; the polyvinyl alcohol may have a molecular weight of about 20,000 Daltons to about 200,000 Daltons; and a weight ratio of the polyvinyl alcohol-polyethylene glycol graft copolymer to the polyvinyl alcohol is 7:3 to 4:6.

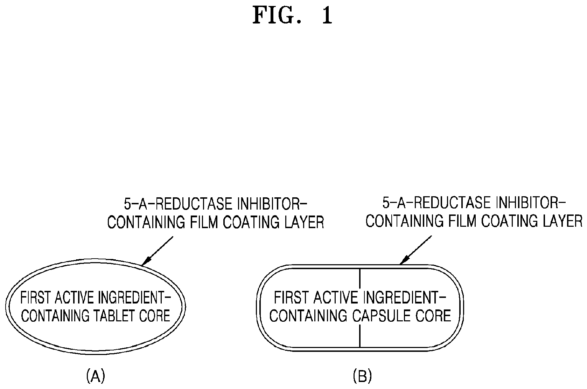

FIG. 1 is a schematic view illustrating composite preparations according to embodiments.

In FIG. 1, (a) is a schematic view illustrating a composite preparation in which a film coating layer containing a 5-α-reductase inhibitor is coated on a surface of a tablet core containing a first active ingredient; and (b) is a schematic view illustrating a composite preparation in which a film coating layer containing a 5-α-reductase inhibitor is coated on a surface of a hard-capsule core containing a first active ingredient.

In some embodiments, the composite preparation may further include an inner coating layer that separates the core and the film coating layer from each other to more effectively prevent interaction between the active ingredients. The inner coating layer may be about 0.01 parts by weight to about 60 parts by weight with respect to 100 parts by weight of the core. For example, a film forming material (a film forming agent and/or coating agent) that may be used for the inner coating layer may be hydroxypropyl methyl cellulose, hydroxypropyl cellulose, hydroxyethyl cellulose, cellulose acetate phthalate, ethyl cellulose, methyl cellulose, polymethacrylate, polyethylene glycol, talc, titanium dioxide, or any mixtures thereof. However, embodiments are not limited thereto. Any film forming materials commonly used to form solid formulations for oral or parenteral administration in the field of pharmaceutics may be used.

In some embodiments, the composite preparation may further include an outer coating layer surrounding the active ingredient-containing film coating layer to further protect the composite preparation from external environments. The outer coating layer may be any coating layer that does not significantly affect a high tensile strength of the active ingredient-containing film coating layer and a high dissolution rate of the active ingredient, which are intended purposes of the composite preparation. Such a coating layer may be obtained by moisture-proofing coating or polishing coating. An appropriate material may be chosen depending on a type of coating by one of ordinary skill in the art based on the technology known in the art.

The outer coating layer may be about 0.01 parts by weight to about 60 parts by weight with respect to 100 parts by weight of the core.

In some embodiments, the composite preparation may further include, in addition to the above-described ingredients, a pharmaceutically acceptable excipient in the core and the 5-α-reductase inhibitor-containing film coating layer. The excipient may be selected from the group consisting of a diluent, a disintegrant, a binder, a stabilizer, a lubricant, and any combination thereof.

The diluent may be selected from the group consisting of microcrystalline cellulose, lactose, Rudy press, mannitol, calcium dihydrogen phosphate, starch, low-substituted hydroxypropyl cellulose, and any combination thereof. However, embodiments are not limited thereto.

The disintegrant may be selected from the group consisting of crospovidone, sodium starch glycolate, croscarmellose sodium, low-substituted hydroxypropyl cellulose, starch, alginic acid, sodium alginate, and any combination thereof. However, embodiments are not limited thereto.

The binder may be selected from the group consisting of hydroxypropyl cellulose, hydroxypropyl methyl cellulose, polyvinylpyrrolidone, copovidone, macrogol, light anhydrous silicic acid, silicate derivatives such as synthetic aluminum silicate, calcium silicate, and magnesium metasilicate aluminate, phosphates such as calcium hydrogen phosphate, carbonates such as calcium carbonate, and any combination thereof. However, embodiments are not limited thereto.

The stabilizer may be selected from the group consisting of alkaline stabilizers, including magnesium carbonate, sodium bicarbonate, sodium carbonate, calcium carbonate, and any combination thereof. However, embodiments are not limited thereto.

The lubricant may be selected from the group consisting of stearic acid, metal salts of stearic acid such as calcium stearate or magnesium stearate, talc, colloid silica, sucrose fatty acid ester, hydrogenated vegetable oil, high-melting point wax, glyceryl fatty acid esters, glycerol dibehenate, and any combination thereof. However, embodiments are not limited thereto.

As used herein, the term "active ingredient" means not only any pharmacological active agent for treatment purpose but also any reagent or any medical agent that may be administered into the human body for diagnostic or preventive purposes. In broader sense, the "active ingredient" may be any material, ingredient, or healthy functional food in a processed form thereof that may be taken for health care purposes to carry out nutrient regulatory functions or physiological functions affecting the body structure and functions.

As used herein, the term "first active ingredient" is used to distinguish an active ingredient used together with a 5-α-reductase inhibitor in the composite preparation, wherein, for ease of description, the active ingredient contained in the core of the composite preparation is referred to as a first active ingredient.

The first active ingredient may be any drugs which need to be administered in combination with the 5-α-reductase inhibitor. For example, the first active ingredient may include tamsulosin or a phosphodiesterase-5 inhibitor (for example, tadalafil, sildenafil, vardenafil, or udenafil).

A combination of 5-α-reductase inhibitor and tamsulosin, and a combination of 5-α-reductase inhibitor and phosphodiesterase-5 inhibitor are known to be advantageous in terms of efficacy enhancement in treating prostatic hypertrophy, due to different mechanisms of the individual active ingredients which are known to be also effective for the treatment of prostatic hypertrophy (EP 1 501 517; BJU Int. 2006 Apr. 97 Suppl 2:39-43; Discussion 44-5). Thus, when including tamsulosin or a phosphodiesterase-5 inhibitor as the first active ingredient, the composite preparation according to any of the embodiments may be used as an effective formulation for treating prostatic hypertrophy.

In some embodiments, the composite preparation may be for oral administration.

In some embodiments, the first active ingredient may be tamsulosin or a pharmaceutically acceptable salt thereof, and the second active ingredient is a 5-α-reductase inhibitor.

The 5-α-reductase inhibitor is selected from the group consisting of finasteride, dutasteride, and any combination thereof. The composite preparation may have a dissolution rate of the 5-α-reductase inhibitor of about 75% or greater in 15 minutes, as evaluated using a Dissolution Test according to the General Tests of the *Korean Pharmacopoeia*

In some embodiments, in consideration of a known daily dose, the composite preparation may include about 0.1 mg to about 0.8 mg, and in some other embodiments, about 0.2 mg to about 0.6 mg of tamsulosin or a pharmaceutically acceptable salt thereof, expressed in terms of the amount of free base, as the first active ingredient, and about 1 mg to about 10 mg of finasteride as the second active ingredient.

In some embodiments, in consideration of a known daily dose, the composite preparation may include about 0.1 mg to about 0.8 mg, and in some other embodiments, about 0.2 mg to about 0.6 mg of tamsulosin or a pharmaceutically acceptable salt thereof, expressed in terms of the amount of free base, as the first active ingredient, and about 0.2 mg to about 0.6 mg of dutasteride as the second active ingredient.

In some embodiments, the first active ingredient may be a phosphodiesterase-5 inhibitor, and the second active ingredient is a 5-α-reductase inhibitor. The phosphodiesterase-5 inhibitor may be tadalafil, sildenafil, vardenafil, udenafil, or any combination thereof. For example, the phosphodiesterase-5 inhibitor may be tadalafil. The tadalafil may be used as a tadalafil free base or a pharmaceutically acceptable salt thereof, for example, hydrobromide, phosphate, sulfate, hydrochloride, maleate, fumarate, lactate, tartrate, citrate, besylate, camsylate, or gluconate. For example, the tadalafil may be a tadalafil free base. However, embodiments are not limited thereto.

The 5-α-reductase inhibitor is selected from the group consisting of finasteride, dutasteride, and any combination thereof. The composite preparation may have a dissolution rate of the 5-α-reductase inhibitor of about 75% or greater in 15 minutes, as evaluated using a Dissolution Test according to the General Tests of the *Korean Pharmacopoeia.*

In some embodiments, in consideration of a known daily dose, the composite preparation may include about 5 mg to about 20 mg, and in some other embodiments, about 5 mg to about 10 mg of tadalafil or a pharmaceutically acceptable salt thereof expressed in terms of the amount of free base, as the first active ingredient, and about 1 mg to about 10 mg of finasteride as the second active ingredient.

In some embodiments, in consideration of a known daily dose, the composite preparation may include about 5 mg to about 20 mg, and in some other embodiments, about 5 mg to about 10 mg of tadalafil or a pharmaceutically acceptable salt thereof, expressed in terms of the amount of free base, as the first active ingredient, and about 0.2 mg to about 0.6 mg of dutasteride as the second active ingredient.

In some embodiments, the composite preparation may include tamsulosin or a pharmaceutically acceptable salt thereof as the first active ingredient, and finasteride as the second active ingredient; the polyvinyl alcohol-polyethylene glycol graft copolymer may include about 65% to about 85% of a polyvinyl alcohol unit and about 15% to about 35% of a polyethylene glycol unit, contain about 0.01 wt% to about 0.5 wt% of colloid silica, and have a weight average molecular weight of about 35,000 Daltons to about 55,000 Daltons; the polyvinyl alcohol may have a molecular weight of about 20,000 Daltons to about 200,000 Daltons; and a weight ratio of the polyvinyl alcohol-polyethylene glycol graft copolymer to the polyvinyl alcohol is 7:3 to 4:6.

A composite preparation according to an embodiment including a phosphodiesterase 5 inhibitor, tamsulosin, or a pharmaceutically acceptable salt thereof as the first active ingredient and a 5-α-reductase inhibitor as the second active ingredient may ensure rapid release and content uniformity of the 5-α-reductase inhibitor, have improved shape and stability due to enhanced tensile strength of the film coating layer. Furthermore, due to the inclusion of the two active ingredients effective for treating prostatic hypertrophy, having different action mechanisms, in a single formulation, the composite preparation may provide a synergistic effect in treating and alleviating prostatic hypertrophy and may increase patient medication compliance.

A composite preparation according to any of the embodiments including a 5-α-reductase inhibitor may be prepared by introducing 5-α-reductase inhibitor-containing film coating layer on a core containing another active ingredient to be administrated in combination with the 5-α-reductase inhibitor, not by simply mixing the composition of individual existing single formulations, and thus may have a remarkably reduced volume. Accordingly, a composite preparation according to any of the embodiments may be formulated as a tablet or capsule having a remarkably reduced size, and consequentially improve patient medication compliance. For example, when a composite preparation is formulated by mixing 183.75 mg of Cialis® as a tadalafil formulation and 153.87 mg of Proscar® as a finasteride formulation, a total mass of the composite preparation may be about 337.62 mg, leading to reduced convenience in taking the composite preparation. However, a composite preparation according to any of the embodiments (for example, Examples 1 to 8) may have a total mass of about 175.5 mg, which is remarkably smaller compared to a composite formulation made by simply combining the compositions of the individual single preparations , and may improve patient medication compliance.

An aspect of the present disclosure provides a method, as defined in claim 10, of preparing a composite preparation according to any of the above-described embodiments, the method including:
preparing the core containing the first active ingredient;
preparing a 5-α-reductase inhibitor-containing coating solution in which the 5-α-reductase inhibitor and the film coating material are dissolved in a mixed solvent of water and 30wt% to 80wt% of an organic solvent; and
coating the core with the 5-α-reductase inhibitor-containing coating solution.

The above-described details of the composite preparations for oral administration according to all the aspects of the embodiments described above may apply to the preparation methods thereof according to embodiments.

In preparing the core, the core may be any core capable of being used as a core in the field of pharmaceutics, in the form of, for example, a tablet, a hard capsule, or a soft capsule. However, embodiments are not limited thereto. In the preparing of the core, any commercially available formulations which may be used as a core may be used, or the core may be directly prepared for use in the invention. The core may be prepared using a known technology in the field of pharmaceutics chosen according to a type of the core by one of one of ordinary skill in the art.

The coating of the core may be performed using any film coating method available used in the field of pharmaceuticals, for example, a pan-coating method, a fluidized-bed coating, or a press-coating method. However, embodiments are not limited thereto.

When the 5-α-reductase inhibitor is finasteride, a typical 5-α-reductase inhibitor known to be teratogenic, a separate independent production line is required to prevent finasteride from being mixed with other medical products during preparation of a finasteride-containing formulation by a pharmaceutical company.. For example, in preparing a solid formulation containing finasteride, all processes, including mixing, granulating, tableting, and coating, must be performed in a segregated and exclusive area. However, a method of preparing a composite preparation according to an embodiment involves applying finasteride with a finasteride-containing coating solution, so that the existing drug preparation facilities may be used just by additionally introducing an exclusive coating facility for finasteride-containing coating, and thus preparing a finasteride formulation may be relatively convenient. Therefore, when using a method of preparing a composite preparation, according to an embodiment, a 5-α-reductase inhibitor-containing composite preparation may be more economically prepared.

### [EXAMPLES]

One or more embodiments of the present disclosure will now be described in detail with reference to the following examples. However, these examples are only for illustrative purposes and are not intended to limit the scope of the one or more embodiments of the present disclosure.

### Examples 1-10 and Comparative Examples 1-8: Preparation of composite formulation including finasteride-containing film coating layer

(Examples 1-8 are not according to the invention)

Coating solutions were prepared by mixing corresponding coating materials with an organic solvent-water mixture solvent in a various mixed ratio according to various compositions presented in Tables 1 to 3, and then coated on tadalafil-containing tablet cores (unit dose mass: 160 mg) by using a pan coater (SFC-30, available from Sejong Pharmatech Co., Ltd.). The tadalafil-containing tablet cores were obtained by mixing tadalafil with mannitol, pregelatinized starch, hydroxypropyl cellulose, sodium lauryl sulfate, and sodium starch glycolate, granulating the resulting mixture, and tableting the resulting granules. The tablets cores coated using the pan coater were dried at about 35°C for about 30 minutes, thereby preparing composite preparations including the tadalafil-containing tablet core coated with finasteride-containing film coating layer. As a result, the prepared composite tablets of Examples 1 to 8 (not according to the invention) were found all to have mass of about 175.5 mg a unit dosage form. Tamsulosin hydrochloride-containing Tamsulosin® capsules (available from Hanmi Pharmaceutical Co., Ltd., Republic of Korea) were coated in the same manner as described above, according to the compositions presented in Table 4. The coated capsules were dried at about 35°C for about 30 minutes, thereby preparing composite preparations including a tamsulosin-containing capsule core coated with a finasteride-containing film coating layer.

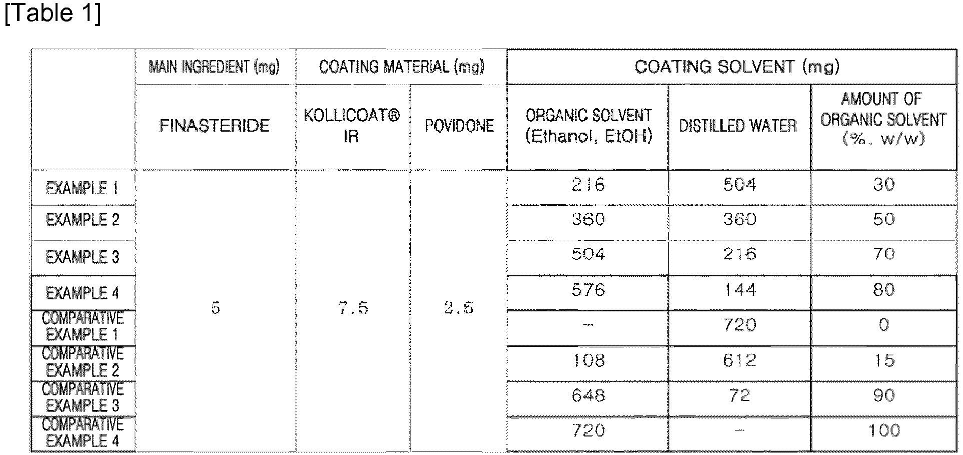

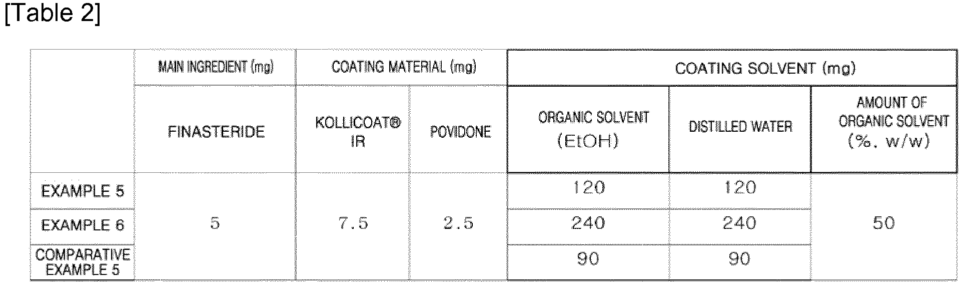

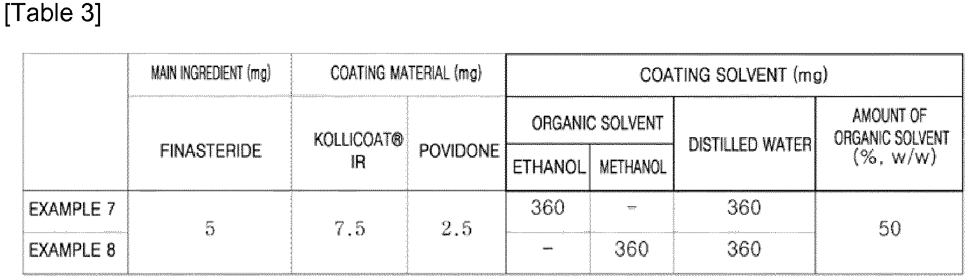

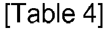

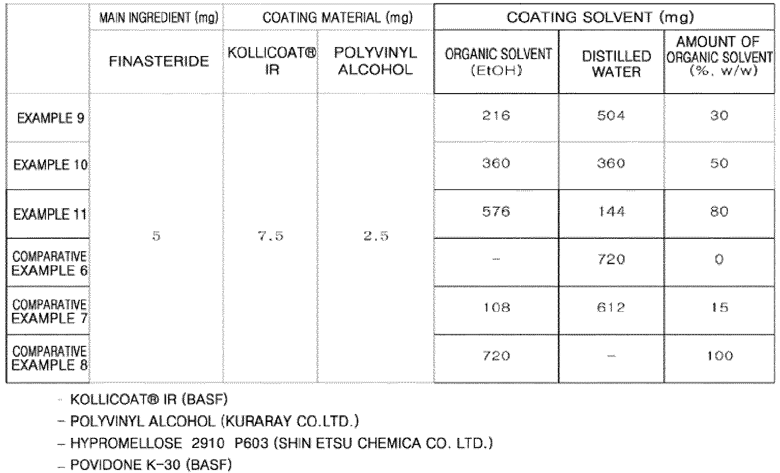

### Test Example 1: Solubility test of finasteride and coating materials

Finasteride, Kollicoat® IR, povidone, and polyvinyl alcohol were added to the organic solvent-water mixture solvents in a variety of ratios presented in Tables 1 to 4, and it was visually observed whether finasteride, Kollicoat® IR, povidone, and polyvinyl alcohol were soluble or not in each of the organic solvent-water mixture solvents. The results are shown in Table 5.

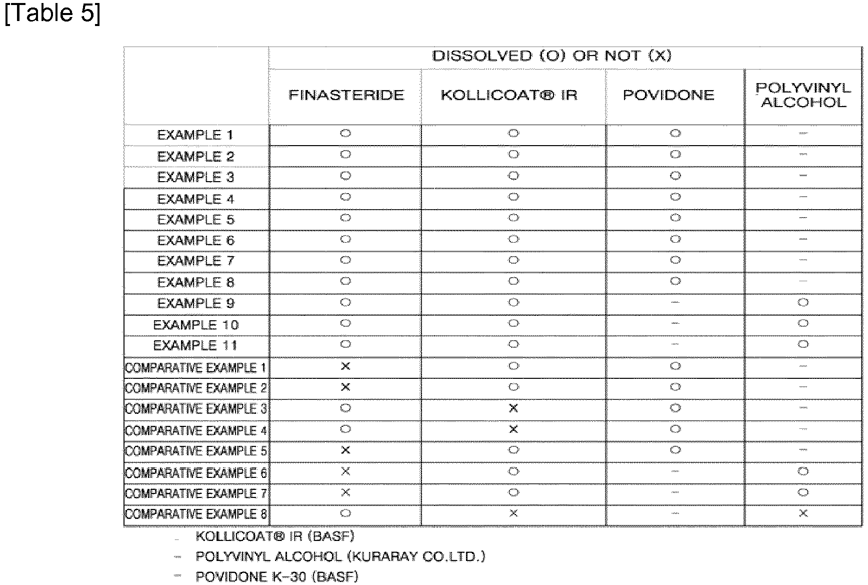

According to the result of observing whether finasteride and the coating materials are soluble in the organic solvent-water mixture solvents in a variety of ratios presented in Tables 1 to 4, povidone was soluble in all of the organic solvent-water mixture solvents in the variety of ratios, while polyvinyl alcohol was soluble in most of the organic solvent-water mixture solvents, except for the organic solvent-water mixture solvent of Comparative Example 8. Finasteride was insoluble in the organic solvent-water mixture solvents of Comparative Example 1, 2, 5, 6, and 7, and Kollicoat® IR was insoluble in the organic solvent-water mixture solvents of Comparative Examples 3, 4, and 8.

### Test Example 2: Dissolution rate confirmation

A dissolution test was performed as follows to evaluate a dissolution rate of finasteride in each of the composite preparations of Examples 1 to 11 and Comparative Examples 1 to 8.

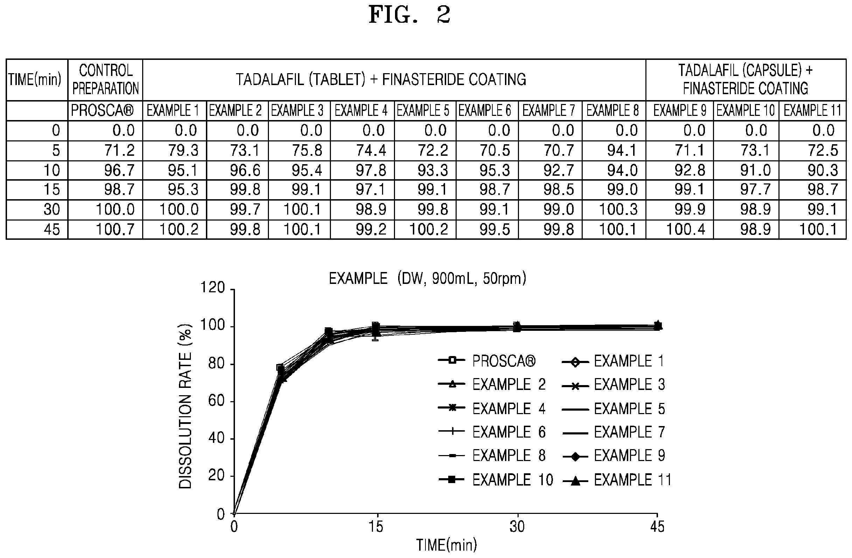

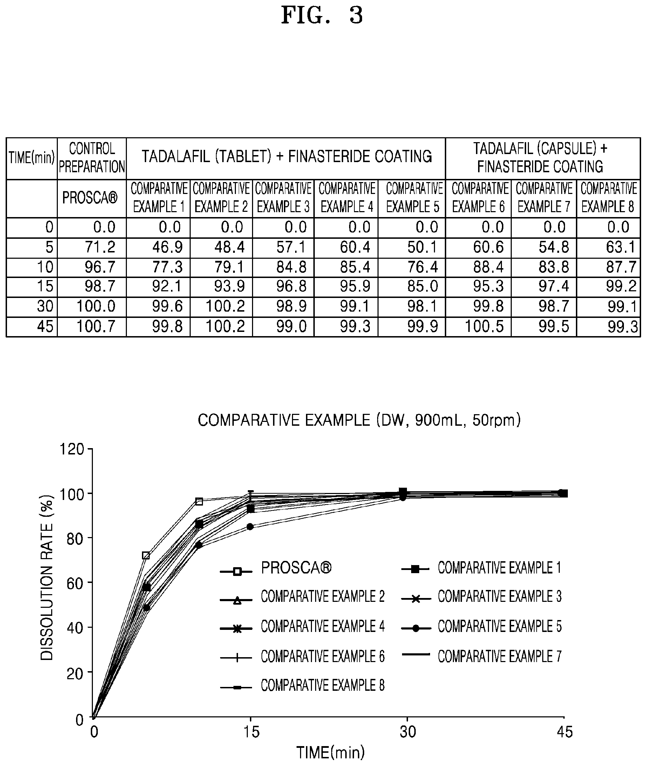

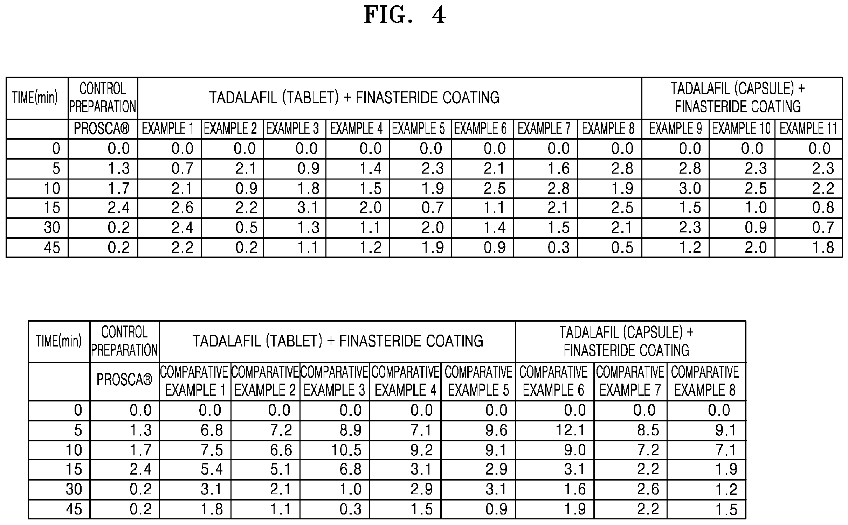

The dissolution test was performed using Paddle method among Dissolution Methods according to the General Tests of the Korean Pharmacopoeia (10th edition). In accordance with the Korean Pharmacopoeia (10th edition), 900 mL of distilled water (DW) was used as a test solution, and the dissolution test was performed at a speed of about 50 rpm according to an operation method for general release formulations. The test solution was taken at 0 minute, 5 minutes, 10 minutes, 15 minutes, 30 minutes, and 45 minutes after start of the test, and analyzed by liquid chromatography according to the General Tests of the Korean Pharmacopoeia (10th edition), thereby obtaining a dissolution rate at each point of time through comparison with a previously prepared standard solution. PROSCA tablets (MSD, 5 mg) were used as a control preparation. The results are shown in FIG. 2 (Examples 1-11) and FIG. 3 (Comparative Examples 1-8). The deviations in the dissolution rates are shown in FIG. 4.

Referring to FIGS. 2 to 4, the composite preparations of Examples 1 to 11 coated using a 30% to 80% (w/w) of an organic solvent were found to have a 5-min dissolution rate of about 70 % to less 80% and a 10-min dissolution rate of about 90 % to less 100%, while the composite preparations of Comparative Examples 1 to 8 had a 5-min dissolution rate of about 40% to about 60 % and a 10-min dissolution rate of about 70% to about 80 %. The composite preparations of Examples 1 to 11 were also found to have a remarkably reduced deviation in the finasteride dissolution rates. Accordingly, as is apparent from the results illustrated in FIGS. 2 to 4, the composite preparations, prepared using a mixed solvent of ethanol and water in a specific ratio able to dissolve both finasteride and the coating materials, were found to have increased dissolution rates of finasteride and reduced deviation in dissolution rates.

As described above, currently commercially available 5-α-reductase inhibitors such as finasteride or dutasteride in the form of immediate release tablets or common tablets have tₘₐₓ of 1 to 2 hours, and thus need to be absorbed fast through high-rate dissolution. Referring to the test results of FIGS. 2 to 4, it was found that when the amount of the organic solvent is out of the range of about 30% to 80%, stable, rapid absorption of finasteride may not be ensured.

### Test Example 3: Confirmation of content uniformity

A content uniformity test was performed to evaluate the content uniformity of finasteride in the composite preparations of Examples 1 to 11 and Comparative Examples 1 to 8.

The content uniformity test was performed using the 'Test for Content Uniformity' as a formulation uniformity test method of the Dissolution Test according to the General Tests of the Korean Pharmacopoeia (10th edition), and evaluation scores were calculated using the 'evaluation core calculation formula.' After a test solution was sampled, and analyzed by liquid chromatography according to the General Tests of the Korean Pharmacopoeia (10th edition), a content uniformity of each composite preparation was calculated through comparison with a previously prepared standard solution. The results are shown in Table 6.

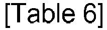

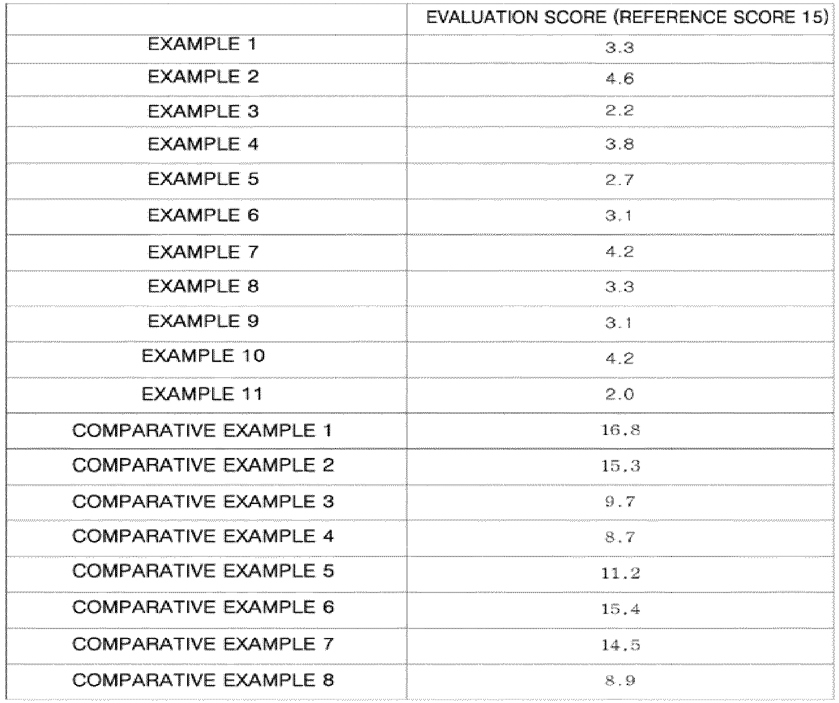

Referring to Table 6, as a result of the content uniformity test, the composite preparations of Examples 1 to 11 prepared using a 30 % to 80 %(w/w) organic solvent were found to have a high content uniformity with an evaluation score less than 5, while the composite preparations of Comparative Examples 1 to 8 had a remarkably lower content uniformity with an evaluation score greater than 8, as compared with the composite preparations of Examples 1 to 11. Many of Comparative Examples 1 to 8 are shown to exceed the reference score for content uniformity.

These results are attributed to the composite preparations of Examples being prepared by coating with the film coating solutions in which finasteride and the coating materials were dissolved, while the composite preparations of Comparative Examples 1 to 8 were prepared by coating with the film coating solutions in which finasteride and the coating materials were dispersed as large particles and not dissolved.

### Test Example 4: Analysis of crystalline form of finasteride-containinq film coating layer

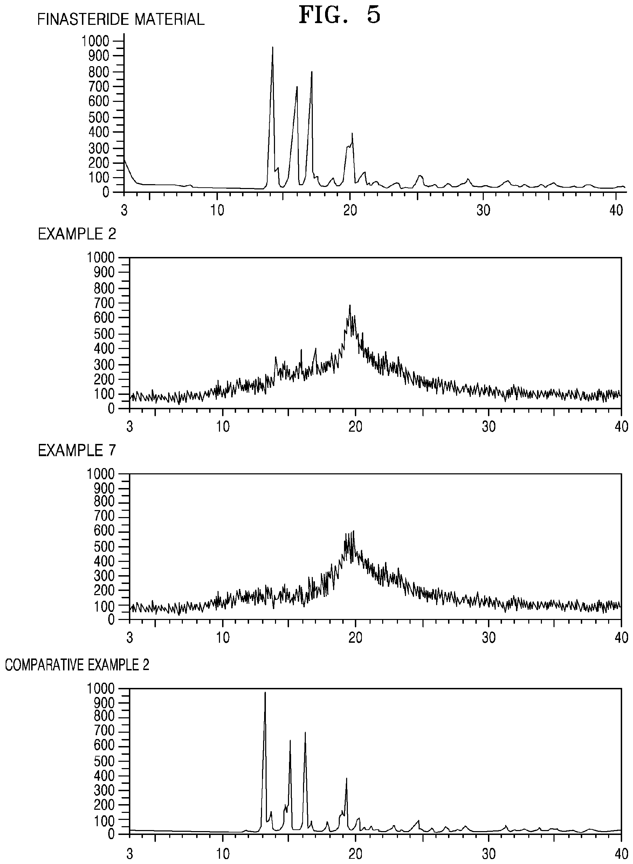

Each film coating solution prepared in Examples 2 and 7 and Comparative Example 2 was sprayed on a Petri dish and dried to form a film. These films were collected, and crystalline forms thereof were analyzed using an X-ray diffractometer (D8 Advance, Bruker) with Cu X-ray radiation at a voltage of 40 kV, at a current of 100 mA, and with a scan rate of 3 deg/min. The results of the X-ray diffractometry of finasteride powder as a raw material, and the dried films prepared using the film coating solutions of Examples 2 and 7 and Comparative Example 2 are shown in FIG. 5.

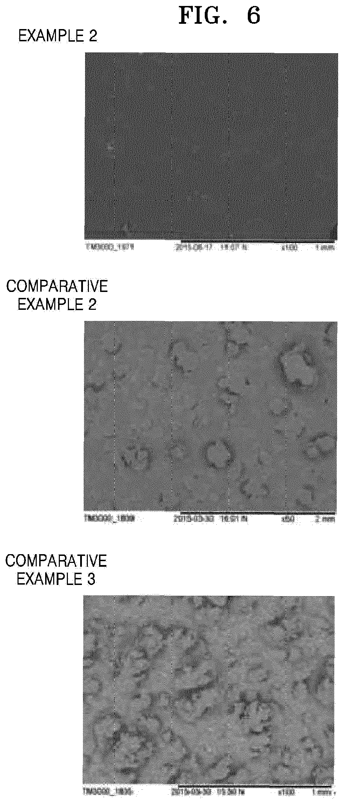

Each of the film coating solutions of Example 2 and Comparative Examples 2 and 3 was sprayed on a Petri dish and dried to form a film. These films were analyzed using an scanning electron microscope (SEM). The resulting SEM images are shown in FIG. 6.

Referring to FIG. 5, the finasteride powder as a raw material and the dried film formed using the film coating solution of Comparative Example 2 were found to be crystalline, while the dried films formed using the film coating solutions of Examples 2 and 7 were found to be amorphous, indicating that the original crystalline structure of finasteride may be changed into an amorphous form in the film coating layers according to embodiments. Referring to FIG. 6, the SEM images also support that finasteride was changed into amorphous form in the film coating layer according to an embodiment, while finasteride in the film coating layers of Comparative Examples remained crystalline.

### Examples 12-21: Preparation of composite preparations including tamsulosin hydrochloride capsule core coated with 5-α-reductase inhibitor-containing film coating layer

(Examples 12 and 17 are not according to the invention)

Coating solutions were prepared by mixing coating materials having different compositions (Examples 12 to 21) as presented in Table 7 with an ethanol-water mixture solution (ethanol: water = 1:1 (v/v)), and then coated on tamsulosin hydrochloride-containing Tamsuloisin® capsules (available from Hanmi Pharmaceutical Co., Ltd., Republic of Korea) by using a pan coater (SFC-30, available from Sejong Pharmatech Co., Ltd.). The prepared capsules were dried at about 35°C for about 30 minutes, thereby preparing composite preparations including a tamsulosin capsule core coated with a 5-α-reductase inhibitor-containing film coating layer.

### Comparative Examples 9-16: Preparation of composite preparations including tamsulosin hydrochloride capsule core coated with 5-α-reductase inhibitor-containing film coating layer

Composite preparations of Comparative Examples 9 to 16 including a tamsulosin capsule core coated with a 5-α-reductase inhibitor-containing film coating layer were prepared in the same manners as in Examples 12 to 21, except that the coating materials were changed according to those of Comparative Examples 9 to 16 presented in Table 7.

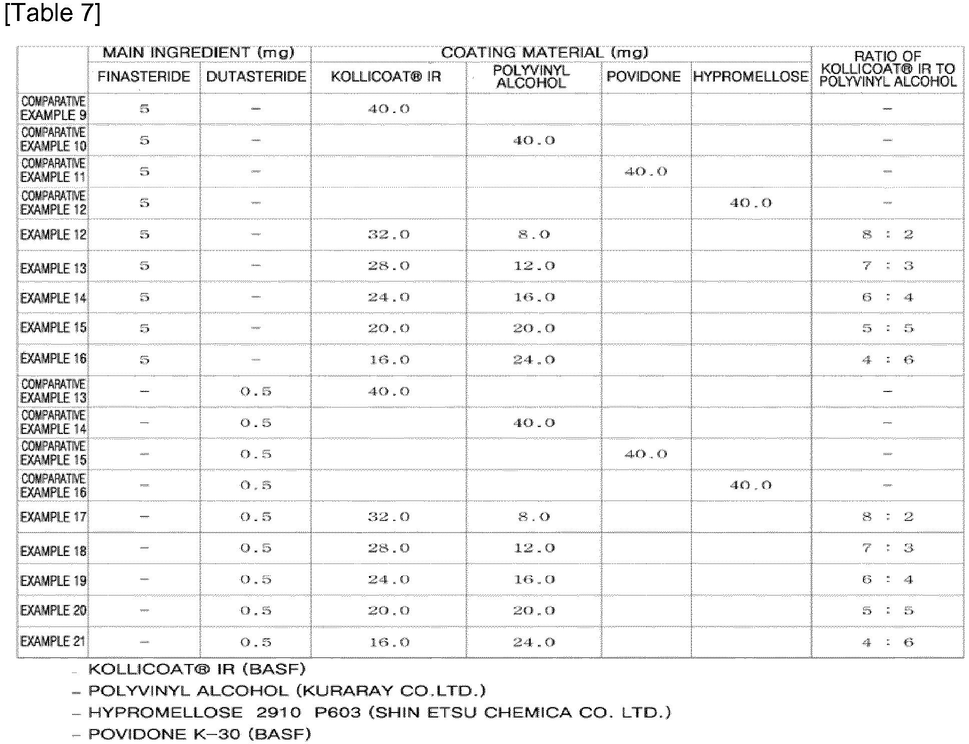

### Examples 22 and 23: Preparation of composite preparations including tamsulosin OD tablet core coated with 5-α-reductase inhibitor-containing film coating layer

Coating solutions were prepared by mixing coating materials having different compositions (Examples 22 and 23) as presented in Table 8 with an ethanol-water mixture solution (ethanol: water = 1:1 (v/v)), and then coated on tamsulosin hydrochloride-containing Tamsuloisin® OD tablets (available from Hanmi Pharmaceutical Co., Ltd., Republic of Korea) by using a pan coater (SFC-30, available from Sejong Pharmatech Co., Ltd.). The prepared composite tablets were dried at about 35°C for about 30 minutes, thereby preparing composite preparations including a Tamsulosin® OD tablet core coated with a 5-α-reductase inhibitor-containing film coating layer.

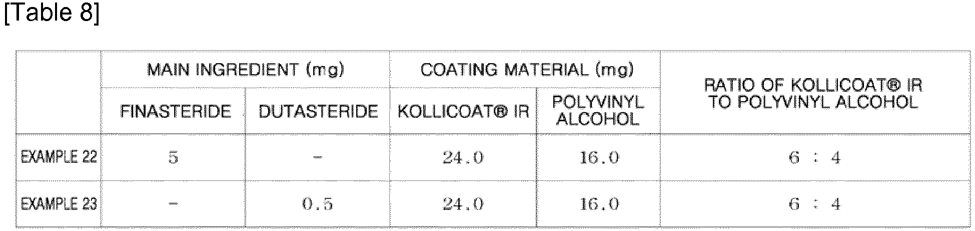

### Test Example 5: Defect test with respect to different coating materials

A defect test of the composite preparations of Comparative Examples 9 to 16 was performed to evaluate film tensile strengths and breakage rates of the composite preparations.

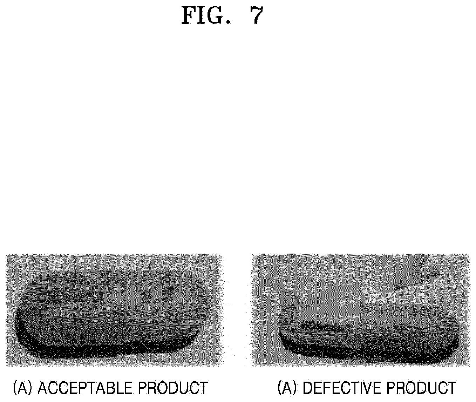

Each composite preparation was packaged using a PTP packing machine (Lab-Blister machine, OMAR FANTASY PLUS), wherein an aluminum mold containing each composite preparation was sealed with an aluminum film. Ten people were randomly chosen and asked to break 100 PTP packages so as to unpack each composite formulation, wherein, as illustrated in FIG. 7, a composite preparation was counted as a defective product when the film coating layer thereof was separated from its core or was broken, or as an acceptable product when the film coating layer remained the same as before packaging, and a percentage of the defective products (hereinafter, referred as a defect rate) was calculated. Photographic images of composite preparations counted as an acceptable product (a) and a defective product (b) are shown in FIG. 7.

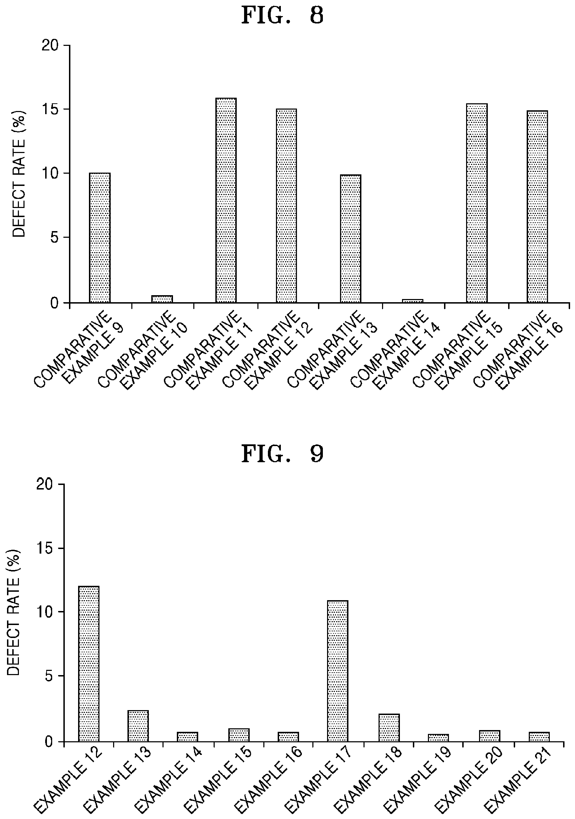

The resulting defect rates of the composite preparations are shown in Table 9 and FIG. 8.

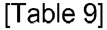

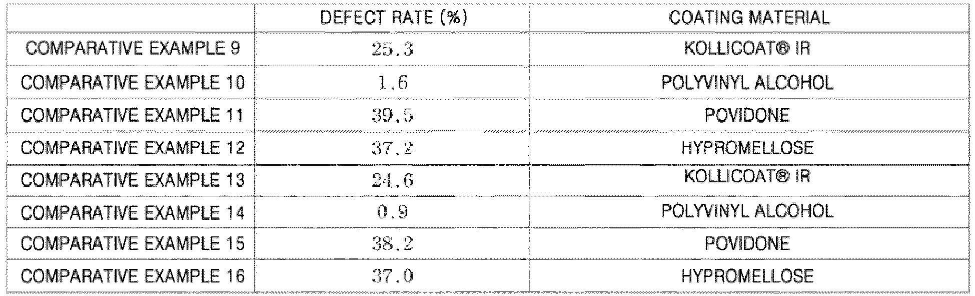

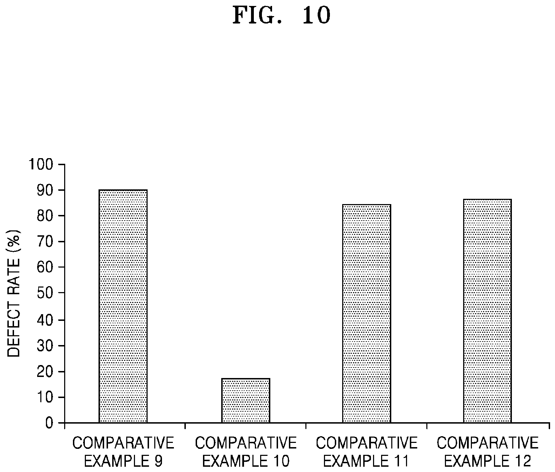

Referring to Table 9 and FIG. 10, the composite preparations of Comparative Examples 9, 11, 12, 13, 15, and 16 using only Kollicoat® IR, povidone, or hypromellose alone as a coating material were found to have damage of the film coating layer with a high defect rate of about 20 % to about 40 %, while the composite preparations of Comparative Examples 10 and 14 using polyvinyl alcohol alone as a coating material were found to have a low defect rate of about 2% or less.

### Test Example 6: Defect test of composite preparations including a combination of polyvinyl alcohol-polyethylene glycol graft copolymer and polyvinyl alcohol as coating material

A defect test of the composite preparations of Examples 12 to 21 was performed in the same manner under the same conditions as those in Test Example 5. The results are shown in Table 10 and FIG. 9.

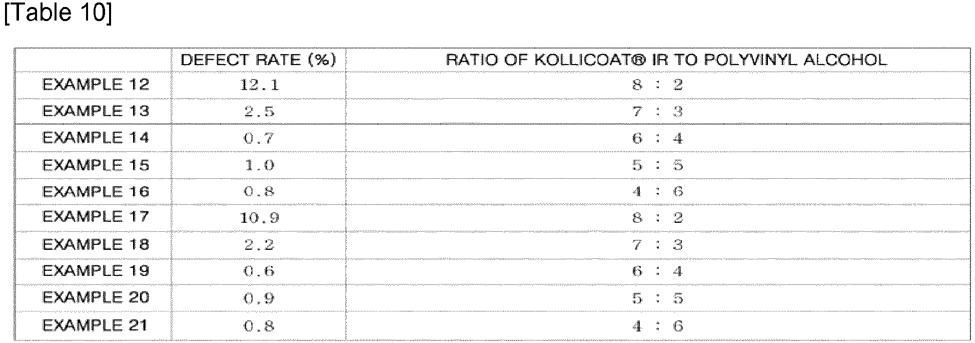

Referring to Tables 9 and 10, the composite preparations of Examples 12 to 21 using a combination of Kollicoat® IR and polyvinyl alcohol as a coating material were found to have a remarkably reduced defect rate as compared with the composite preparations of Comparative Examples 9 and 13 using Kollicoat® IR alone as a coating material.

In particular, the composite preparations of Examples 13 to 16 and Examples 18 to 21 using Kollicoat® IR and polyvinyl alcohol in a ratio of about 7:3 to about 4:6 were found to have a lower defect rate as compared with the composite preparations of Examples 12 and 17 using Kollicoat® IR and polyvinyl alcohol in a ratio of about 8:2.

### Test Example 7: Defect test under stress conditions with respect to different coating materials

A defect test of the composite preparations of Comparative Examples 9 to 12 was performed in the same manner as in Test Example 5 after storage in a thermostatic chamber at about 60°C and at a relative humidity (RH) of 0% for one week, to evaluate stability of each composite preparation with respect to storage time. The test results are shown in Table 11 and FIG. 10.

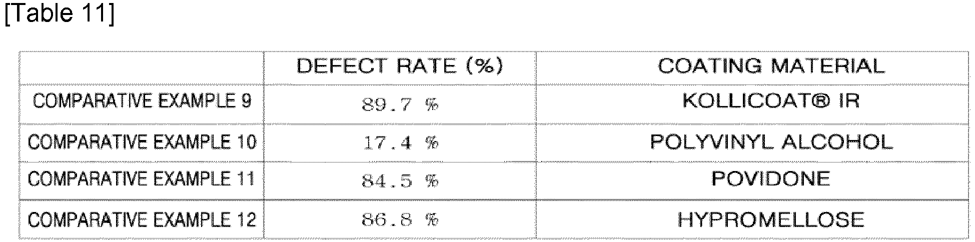

Referring to Table 11 and FIG. 10, the composite preparations of Comparative Examples 9, 11, and 12 using Kollicoat® IR, povidone or hypromellose alone as a coating material were all found to have a high defect rate of about 80% or greater. However, the composite preparation of Comparative Example 10 using polyvinyl alcohol as a coating material was found to have a defect rate of about 17.4%, exhibiting an improved shape and improved stability as compared with the composite preparations of Comparative Examples 9, 11, and 12.

### Test Example 8: Dissolution test with respect to different coating materials

A dissolution test was performed as follows to evaluate a dissolution rate of finasteride in each of the composite preparations of Comparative Examples 9 to 12.

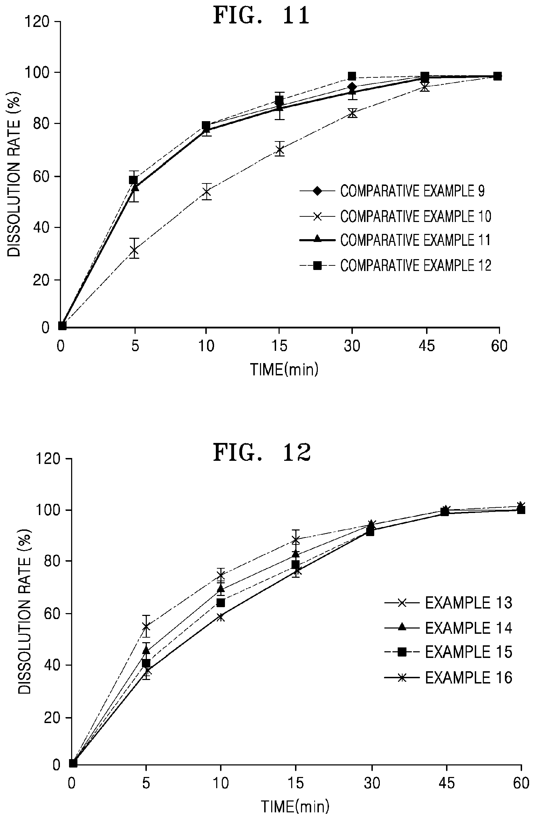

The dissolution test was performed using Dissolution Method II (Paddle method) according to the General Tests of the Korean Pharmacopoeia (10th edition). In accordance with the Korean Pharmacopoeia (10th edition), 900 mL of distilled water was used as a test solution, and the dissolution test was performed at a speed of about 50 rpm according to an general operation method for release formulations. The test solution was taken at 0 minutes, 5 minutes, 10 minutes, 15 minutes, 30 minutes, 45 minutes, and 60 minutes after beginning the test, and analyzed by liquid chromatography according to the General Tests of the Korean Pharmacopoeia (10th edition), thereby obtaining a dissolution rate at each point of time through comparison with a previously prepared standard solution. The results are shown in Table 12 and FIG. 11.

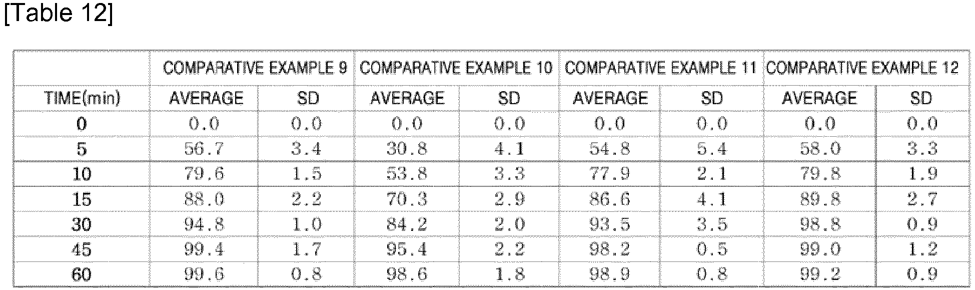

Referring to Table 12 and FIG. 11, the composite preparations of Comparative Examples 9, 11, and 12 using Kollicoat® IR, povidone, or hypromellose alone, were found to have a relatively high dissolution rate as compared with the composite preparation of Comparative Example 10 using polyvinyl alcohol.

Finasteride is a drug which requires a high dissolution rate to achieve high bioavailability. Accordingly, the composite preparation of Comparative Example 10 using polyvinyl alcohol may have lower bioavailability as compared with the composite preparations of Comparative Examples 9, 11, and 12.

### Test Example 9: Dissolution test of composite preparations including a combination of polyvinyl alcohol-polyethylene glycol graft copolymer and polyvinyl alcohol as coating material

A dissolution test of the composite preparations of Examples 13 to 16 was performed under the same conditions as those in Test Example 8. The results are shown in Table 13 and FIG. 12.

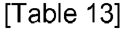

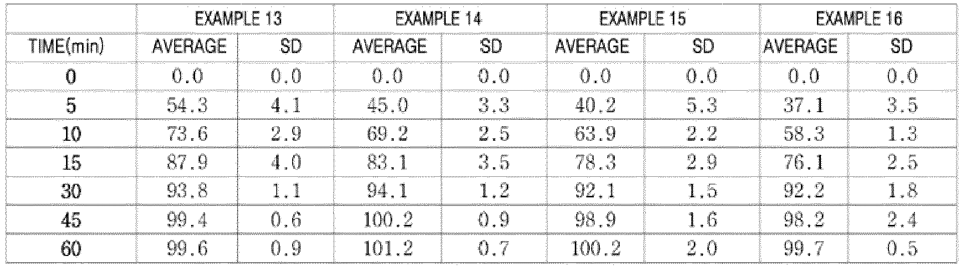

Referring to Table 13 and FIG. 12, the composite preparations of Examples 13 to 16 using Kollicoat® IR and polyvinyl alcohol in a weight ratio of about 7:3 to about 4:6 were found to have a dissolution rate of about 75% or greater in 15 minutes.

### Test Example 10: Defect test with respect to core type of composite preparation including a combination of polyvinyl alcohol-polyethylene glycol graft copolymer and polyvinyl alcohol as coating material

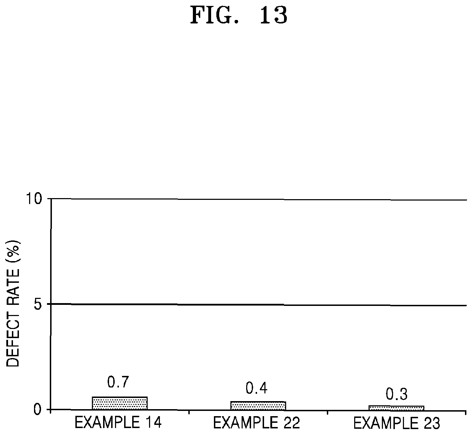

A defect test of the composite preparations of Examples 14, 22, and 23 was performed in the same manner and under the same conditions as those in Test Example 5. The results are shown in Table 14 and FIG. 13.

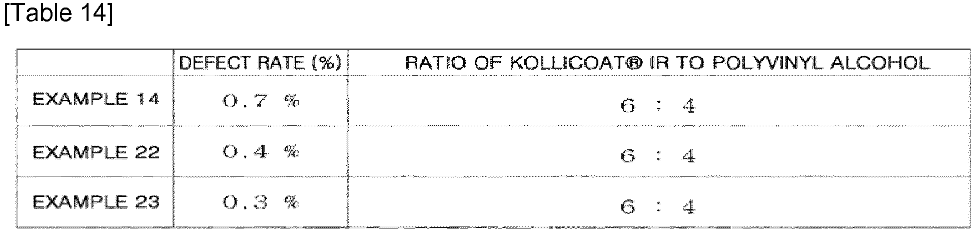

As a result of the defect test, the composite preparations of Examples 22 and 23 using a tamsulosin tablet core and the same coating material as the composite preparation of Example 14 (using tamsulosin capsule core), which was found to have an improved coating shape and stability with a high dissolution rate, were also found to have a low defect rate of about 1% or less. Therefore, the second active ingredient -containing film coating layer of a composite preparation according to any of the embodiments was found to be applicable to various core types, not only to hard capsule cores.

While this invention has been particularly shown and described with reference to preferred embodiments thereof, it will be understood by those skilled in the art that various changes in form and details may be made therein without departing from the scope of the invention as defined by the appended claims. The disclosed embodiments should be considered in descriptive sense only and not for purposes of limitation. Therefore, the scope of the invention is defined not by the detailed description of the invention but by the appended claims, and all differences within the scope will be construed as being included in the present invention.

## Claims

1. A composite preparation comprising:
a core containing a first active ingredient; and
a film coating layer containing a 5-α-reductase inhibitor,
wherein the film coating layer is formed by coating with a film coating solution comprising the 5-α-reductase inhibitor and a film coating material in a mixed solvent of water and 30wt% to 80wt% of an organic solvent with respect to a total weight of the mixed solvent,
wherein the film coating material is a combination of a polyvinyl alcohol-polyethylene glycol graft copolymer and polyvinyl alcohol,
wherein a weight ratio of the polyvinyl alcohol-polyethylene glycol graft copolymer to the polyvinyl alcohol is 7:3 to 4:6, and
wherein the 5-α-reductase inhibitor is selected from the group consisting of finasteride, dutasteride, and any combination thereof.

2. The composite preparation of claim 1, wherein the core is a tablet, a hard capsule, or a soft capsule.

3. The composite preparation of claim 1, wherein the organic solvent is methanol, ethanol, acetone, chloroform, dimethyl sulfoxide (DMSO), or any combination thereof.

4. The composite preparation of claim 1, wherein the polyvinyl alcohol has a molecular weight of 20,000 Daltons to 200,000 Daltons.

5. The composite preparation of claim 1, wherein the polyvinyl alcohol-polyethylene glycol graft copolymer consists of 65% to 85% of a polyvinyl alcohol unit and 15% to 35% of a polyethylene glycol unit, contains 0.01 wt% to 0.5 wt% of colloid silica, and has a weight average molecular weight of 35,000 Daltons to 55,000 Daltons; the polyvinyl alcohol has molecular weight of 20,000 Daltons to 200,000 Daltons.

6. The composite preparation of claim 1, wherein the first active ingredient comprises tamsulosin, a phosphodiesterase-5-inhibitor, or a pharmaceutically acceptable salt thereof.

7. The composite preparation of claim 1, wherein the first active ingredient comprises 5 mg to 20 mg of tadalafil or a pharmaceutically acceptable salt thereof, expressed in terms of the amount of free base, and the 5-α-reductase inhibitor comprises 1 mg to 10 mg of finasteride.

8. The composite preparation of claim 7, wherein an amount of the organic solvent is 120 mg to 576 mg per 5 mg of finasteride.

9. The composite preparation of claim 1, wherein the composite preparation has a 5-α-reductase inhibitor dissolution rate of 75% or greater in 15 minutes, as evaluated using a Dissolution Test according to the General Tests of the *Korean Pharmacopoeia.*

10. A method of preparing a composite preparation according to any one of claims 1 to 9, the method comprising:
preparing the core containing the first active ingredient;
preparing a 5-α-reductase inhibitor-containing coating solution in which the 5-α-reductase inhibitor and a film coating material are dissolved in an mixed solvent of water and 30wt% to 80wt% of an organic solvent based on a total weight of the mixed solvent; and
coating the core with the 5-α-reductase inhibitor-containing coating solution,
wherein the film coating material is a combination of a polyvinyl alcohol-polyethylene glycol graft copolymer and polyvinyl alcohol,
wherein a weight ratio of the polyvinyl alcohol-polyethylene glycol graft copolymer to the polyvinyl alcohol is 7:3 to 4:6, and
wherein the 5-α-reductase inhibitor is selected from the group consisting of finasteride, dutasteride, and any combination thereof.

## Patentansprüche

1. Verbundstoffpräparat, umfassend:
einen Kern, der einen ersten Wirkstoff enthält; und
eine Filmbeschichtungsschicht, die einen 5-α-Reduktasehemmer enthält,
wobei die Filmbeschichtungsschicht durch Beschichten mit einer Filmbeschichtungslösung gebildet wird, umfassend den 5-α-Reduktasehemmer und ein Filmbeschichtungsmaterial in einem gemischten Lösemittel aus Wasser und 30 Gew.-% bis 80 Gew.-% eines organischen Lösemittels, bezogen auf das Gesamtgewicht des gemischten Lösemittels,
wobei das Filmbeschichtungsmaterial eine Kombination aus einem Polyvinylalkohol-Polyethylenglykol-Pfropfcopolymer und einem Polyvinylalkohol ist,
wobei ein Gewichtsverhältnis des Polyvinylalkohol-Polyethylenglykol-Pfropfcopolymers zu dem Polyvinylalkohol 7:3 bis 4:6 beträgt, und
wobei der 5-α-Reduktasehemmer ausgewählt ist aus der Gruppe bestehend aus Finasterid, Dutasterid und einer beliebigen Kombination davon.

2. Verbundstoffpräparat nach Anspruch 1, wobei der Kern eine Tablette, eine Hartkapsel oder eine Weichkapsel ist.

3. Verbundstoffpräparat nach Anspruch 1, wobei das organische Lösemittel Methanol, Ethanol, Aceton, Chloroform, Demethylsulfoxid (DMSO) oder eine beliebige Kombination davon ist.

4. Verbundstoffpräparat nach Anspruch 1, wobei der Polyvinylalkohol ein Molekulargewicht von 20.000 Dalton bis 200.000 Dalton aufweist.

5. Verbundstoffpräparat nach Anspruch 1, wobei das Polyvinylalkohol-Polyethylenglykol-Pfropfcopolymer zu 65% bis 85% aus einer Polyvinylalkohol-Einheit und zu 15% bis 35% einer Polyethylenglykol-Einheit besteht, 0,01 Gew.-% bis 0,5 Gew.-% kolloidales Siliciumdioxid enthält, und ein Gewichtsmittel des Molekulargewichts von 35.000 Dalton bis 55.000 Dalton aufweist; der Polyvinylalkohol ein Molekulargewicht von 20.000 Dalton bis 200.000 Dalton aufweist.

6. Verbundstoffpräparat nach Anspruch 1, wobei der erste Wirkstoff Tamsulosin, einen Phosphodiesterase-5-Hemmer oder ein pharmazeutisch verträgliches Salz davon umfasst.

7. Verbundstoffpräparat nach Anspruch 1, wobei der erste Wirkstoff 5 mg bis 20 mg Tadalafil oder ein pharmazeutisch verträgliches Salz davon umfasst, ausgedrückt in Form der Menge an freier Base, und der 5-α-Reduktasehemmer 1 mg bis 10 mg Finasterid umfasst.

8. Verbundstoffpräparat nach Anspruch 7, wobei eine Menge des organischen Lösemittels 120 mg bis 576 mg pro 5 mg Finasterid beträgt.

9. Verbundstoffpräparat nach Anspruch 1, wobei das Verbundstoffpräparat eine Auflösungsgeschwindigkeit des 5-α-Reduktasehemmers von 75% oder größer in 15 Minuten aufweist, was unter Anwendung eines Auflösungstests gemäß den "General Tests of the *Korean Pharmacopoeia"* evaluiert wird.

10. Verfahren zum Herstellen eines Verbundstoffpräparats nach einem der Ansprüche 1 bis 9, wobei das Verfahren umfasst:
Herstellen des Kerns, der den ersten Wirkstoff enthält;
Herstellen einer 5-α-Reduktasehemmer enthaltenden Beschichtungslösung, in der der 5-α-Reduktasehemmer und ein Filmbeschichtungsmaterial in einem gemischten Lösemittel aus Wasser und 30% bis 80% eines organischen Lösemittels, basierend auf einem Gesamtgewicht des gemischten Lösemittels, aufgelöst sind; und
Beschichten des Kerns mit der 5-α-Reduktasehemmer enthaltenden Beschichtungslösung,
wobei das Filmbeschichtungsmaterial eine Kombination aus Polyvinylalkohol-Polyethylenglykol-Pfropfcopolymer und Polyvinylalkohol ist,
wobei ein Gewichtsverhältnis des Polyvinylalkohol-Polyethylenglykol-Pfropfcopolymers zu dem Polyvinylalkohol 7:3 bis 4:6 beträgt, und
wobei der 5-α-Reduktasehemmer ausgewählt ist aus der Gruppe bestehend aus Finasterid, Dutasterid und einer beliebigen Kombination davon.

## Revendications

1. Préparation composite, comprenant:
un cœur contenant un premier ingrédient actif; et
une couche de revêtement pelliculaire contenant un inhibiteur de la 5-α-réductase,
dans laquelle la couche de revêtement pelliculaire est formée par le revêtement avec une solution de revêtement pelliculaire comprenant l'inhibiteur de 5-α-réductase et une matière de revêtement pelliculaire dans un solvant mixte d'eau et de 30% en poids à 80% en poids d'un solvant organique par rapport à un poids total du solvant mixte,
dans laquelle la matière de revêtement pelliculaire est une combinaison d'un copolymère greffé alcool polyvinylique-polyéthylèneglycol et d'alcool polyvinylique,
dans laquelle un rapport pondéral du copolymère greffé alcool polyvinylique-polyéthylèneglycol à l'alcool polyvinylique est de 7:3 à 4:6, et
dans laquelle l'inhibiteur de 5-α-réductase est choisi dans le groupe constitué par le finastéride, le dutastéride, et toute combinaison de ceux-ci.

2. Préparation composite selon la revendication 1, dans laquelle le cœur est un comprimé, une capsule dure ou une capsule molle.

3. Préparation composite selon la revendication 1, dans laquelle le solvant organique est le méthanol, l'éthanol, l'acétone, le chloroforme, le diméthylsulfoxyde (DMSO), ou toute combinaison de ceux-ci.

4. Préparation composite selon la revendication 1, dans laquelle l'alcool polyvinylique a un poids moléculaire de 20000 daltons à 200000 daltons.

5. Préparation composite selon la revendication 1, dans laquelle le copolymère greffé alcool polyvinylique-polyéthylèneglycol consiste en 65% à 85% d'un motif alcool polyvinylique et 15% à 35% d'un motif polyéthylèneglycol, contient 0,01% à 0,5% en poids de silice colloïdale, et a un poids moléculaire moyen en poids de 35000 daltons à 55000 daltons; l'alcool polyvinylique a un poids moléculaire de 20000 daltons à 200000 daltons.

6. Préparation composite selon la revendication 1, dans laquelle le premier ingrédient actif comprend la tamsulosine, un inhibiteur de la phosphodiestérase-5, ou un sel pharmaceutiquement acceptable de ceux-ci.

7. Préparation composite selon la revendication 1, dans laquelle le premier ingrédient actif comprend 5 mg à 20 mg de tadalafil ou d'un sel pharmaceutiquement acceptable de celui-ci, exprimé en termes de la quantité de base libre, et l'inhibiteur de 5-α-réductase comprend 1 mg à 10 mg de finastéride.

8. Préparation composite selon la revendication 7, dans laquelle une quantité du solvant organique est de 120 mg à 576 mg pour 5 mg de finastéride.

9. Préparation composite selon la revendication 1, laquelle préparation composite a un taux de dissolution de l'inhibiteur de 5-α-réductase de 75% ou plus en 15 minutes, tel qu'évalué en utilisant un Test de Dissolution selon les Tests Généraux de la pharmacopée coréenne.

10. Procédé pour préparer une préparation composite selon l'une quelconque des revendications 1 à 9, le procédé comprenant:
la préparation du cœur contenant le premier ingrédient actif;
la préparation d'une solution de revêtement contenant l'inhibiteur de 5-α-réductase dans laquelle l'inhibiteur de 5-α-réductase et une matière de revêtement pelliculaire sont dissous dans un solvant mixte d'eau et de de 30% en poids à 80% en poids d'un solvant organique sur la base d'un poids total du solvant mixte; et
le revêtement du cœur avec la solution de revêtement contenant l'inhibiteur de 5-α-réductase;
dans lequel la matière de revêtement pelliculaire est une combinaison d'un copolymère greffé alcool polyvinylique-polyéthylèneglycol et d'alcool polyvinylique,
dans lequel un rapport pondéral du copolymère greffé alcool polyvinylique-polyéthylèneglycol à l'alcool polyvinylique est de 7:3 à 4:6, et
dans laquelle l'inhibiteur de 5-α-réductase est choisi dans le groupe constitué par le finastéride, le dutastéride, et toute combinaison de ceux-ci.
